# EUROPÄISCHE PATENTSCHRIFT

(11) **EP 1 955 103 B1**
(45) Veröffentlichungstag und Bekanntmachung des Hinweises auf die Patenterteilung: **02.05.2018**
(21) Anmeldenummer: 07723551.3
(22) Anmeldetag: 23.03.2007
(51) Int. Cl.: G02B 21/00, G02B 21/08, A61B 3/13, A61B 3/117, A61B 3/00, G02B 21/06, G02B 21/22, A61B 90/30, G02B 27/00, G02B 21/18, A61B 90/00

(54) **Opthalmologisches Operationsmikroskop**
Microscope for ophthalmologic surgery
Microscope pour chirurgie ophthalmologique

(30) Priorität: 24.03.2006 DE 102006013761
(43) Veröffentlichungstag der Anmeldung: 13.08.2008
(73) Patentinhaber: Carl Zeiss Meditec AG, 07745 Jena (DE)
(72) Erfinder: MERZ, Franz, 73433 Aalen (DE); REIMER, Peter, 73479 Ellwangen (DE); STRÄHLE, Fritz, 73540 Heubach (DE)
(74) Vertreter: Müller, Thomas
(86) Internationale Anmeldenummer: PCT/EP2007/002603
(87) Internationale Veröffentlichungsnummer: WO 2007/110207

(56) Entgegenhaltungen:
- WO-A-02/26121
- DE-A1- 19 824 460
- US-A- 6 003 993
- HARRY PAUL, ED.: "Lexikon der Optik, Bd M bis Z" 2003, SPRINGER AKADEMISCHER VERLAG GMBH , HEIDELBERG , XP002439491 Abbildungen 1-3 Seite 33, linke Spalte, Absätze AUFLICHT,HELLFELD

## Beschreibung

Die vorliegende Erfindung betrifft ein ophthalmologisches Operationsmikroskop gemäß dem Oberbegriff von Patentanspruch 1.

Insbesondere kann das Operationsmikroskop als ophthalmologisches Operationsmikroskop ausgebildet sein, das beispielsweise für eine spezielle Anwendung in der Augenchirurgie eingesetzt wird, nämlich der Kataraktchirurgie.

Bei der Kataraktchirurgie wird eine - beispielsweise durch den grauen Star getrübte - Augenlinse durch eine Kunstlinse ersetzt.

Die Augenlinse eines Auges befindet sich in einer dünnen Umhüllung, der so genannten Linsenkapsel. Zur Entfernung der Augenlinse wird durch einen dünnen Schnitt in die Linsenkapsel ein Zugang zur Augenlinse geschaffen und die Augenlinse mit einem mikrochirurgischen Gerät zunächst in kleine Einzelstücke zerteilt, die dann mittels einer Absaugvorrichtung entfernt werden.

Dieser Vorgang findet unter mikroskopischer Beobachtung - beispielsweise unter stereomikroskopischer Beobachtung - unter Einsatz einer für solche Eingriffe speziell ausgelegten Beleuchtungseinrichtung statt. Diese Beleuchtungseinrichtung stellt sowohl eine für die Ausleuchtung des gesamten Operationsfelds notwendige Umfeldbeleuchtung als auch eine für die Kataraktoperation entscheidend wichtige rote Hintergrundbeleuchtung für das eigentliche auf den Pupillenbereich der Augenlinse begrenzte Operationsfeld dar. Diese rote Hintergrundbeleuchtung rührt von dem Anteil des Beleuchtungslichts her, der über die transparenten Augenmedien schließlich auf die wegen einer guten Durchblutung rot erscheinenden Netzhaut trifft, von dieser reflektiert wird und dann natürlich über das Operationsmikroskop auch vom Chirurgen als rot erscheinende Hintergrundbeleuchtung beobachtet werden kann. Diese in der Kataraktchirurgie ganz charakteristische rote Hintergrundbeleuchtung ist in Fachkreisen allgemein unter dem Begriff "roter Reflex" bekannt.

Für eine optimale Erkennung der für die Kataraktoperation relevanten Details erweist sich für den Operateur eine möglichst homogene rote Hintergrundbeleuchtung als eine notwendige Voraussetzung. Eine erste Anforderung an die Beleuchtungseinrichtung ist also eine möglichst gute Homogenität des roten Reflexes über die gesamte Patientenpupille zu gewährleisten.

Zur vollständigen Beseitigung der Linsenreste der in winzige Teilstücke zerkleinerten Augenlinse und zur guten Erkennung von durchsichtigen Membranen, beispielsweise von der Linsenkapsel, muss eine weitere Anforderung erfüllt werden, nämlich eine gute Kontrastierung von Phasenobjekten und zwar möglichst auch über die gesamte Patientenpupille.

Für die Operation am Auge, und hier insbesondere bei Kataraktoperationen, wird ein homogener, heller "roter Reflex" und eine gute Kontrastierung der Phasenobjekte über den gesamten Bereich der Patienten-Augenpupille gefordert.

Die bisher verwendeten Operationsmikroskope erfüllen diese Anforderungen für mehr oder weniger große Bereiche der Augenpupille. Es muss immer ein Kompromiss zwischen den Hauptanforderungen guter, homogener "roter Reflex" und gute Kontrastierung der Phasenobjekte eingegangen werden.

Zumeist wird unter einem kleinen Winkel zur Beobachtung beleuchtet. Dies hat jedoch zur Folge, dass der "rote Reflex" über die Patientenpupille nicht gleichmäßig hell erscheint. Als günstig hat sich bisher ein Beleuchtungswinkel zwischen 2 und 4 Grad bewährt. Bei diesem Winkel erhält man einen guten Kompromiss zwischen guter Kontrastierung und Ausleuchtung der Patientenpupille. Der "rote Reflex" reagiert aber bei dieser Anordnung empfindlich auf ein Verrollen des Patientenauges während der Operation. Weitere Probleme können bei kleinen Patientenpupillen oder im Zusammenhang mit der Refraktion des Patientenauges auftreten.

Versuche mit einer Koaxial-Beleuchtung führten zwar zu einem guten, homogenen "roten Reflex", aber zu einer schlechten Kontrastierung der Phasenobjekte und haben sich in der Praxis daher bisher nicht bewährt. Dabei war die Beleuchtungsoptik derart angeordnet, dass ein Beleuchtungsspiegel (oder Prisma) zwischen den beiden Strahlengängen des Stereomikroskops lag. Es handelte sich dabei also nicht um eine exakte 0°-Beleuchtung, die genau aus derselben Richtung erfolgt wie die Beobachtung.

Eine Möglichkeit, einen homogenen hellen "roten Reflex" bei gleichzeitig guter Kontrastierung zu erzielen, lässt sich dadurch erreichen, dass der Beleuchtungsstrahl in zwei Teilstrahlbündel aufgeteilt wird. Dabei wird jedes Teilstrahlbündel so geführt, dass für den linken und den rechten Beobachtungsstrahlengang eines (Stereo-)Operationsmikroskops das zu beobachtende Objekt, beispielsweise ein Auge, aus derselben Richtung beleuchtet wird, aus der auch die Beobachtung erfolgt (0°-Beleuchtung). Eine derartige Lösung ist beispielsweise in der älteren Anmeldung DE 10 2004 050 651 A1 der Anmelderin beschrieben.

Ein generelles Problem bei solchen "Koaxial-Lösungen" ist, dass an optischen Grenzflächen, und hier insbesondere am Hauptobjektivelement des Stereomikroskops, störende Reflexe auftreten können, welche die Beobachtung des Operationsfeldes negativ beeinträchtigen, etwa durch Aufhellungen im Sehfeld.

Bei den bisher gebräuchlichen Beleuchtungs- beziehungsweise Beobachtungssystemen wurde in der Regel bisher auf eine koaxiale Beleuchtungsanordnung, trotz deren Vorteilen für den "roten Reflex" verzichtet, oder aber es wurden bisher andere Maßnahmen ergriffen, die aber eine Reihe von Nachteilen aufweisen:
Ein Ausblenden der Reflexe im Beobachtungsstrahlengang führt beispielsweise zu Vignettierungen beziehungsweise Bildausfällen. Eine stärkere Dezentrierung des Hauptobjektivs relativ zu den Beobachtungsstrahlbündeln erfordert beispielsweise deutlich größere und damit auch teurere Optiken. Weiterhin wird die Korrektur der dadurch auftretenden prismatischen Farbfehler aufwändiger. Eie Reflexunterdrückung könnte beispielsweise auch durch Verwendung von polarisiertem Licht erreicht werden. Durch einen solchen Lösungsansatz geht jedoch mehr als die Hälfte des Lichts in der Beleuchtung verloren (durch den Polarisator). Zum anderen wird noch einmal mehr als die Hälfte des vom Objekt zurückgestrahlten Lichts in dem Analysator zurückgehalten. Insbesondere der letztgenannte Punkt ist bei Anwendungen am Auge nachteilig, da mit entsprechend größeren Intensitäten beleuchtet werden muss.

Neben konventionellen Mikroskopen sind auch konfokale Mikroskope bekannt. Zu nennen sind hierzu beispielsweise die US 6,003,93 A oder die DE 198 24 460 A1.

Ausgehend vom genannten Stand der Technik liegt der vorliegenden Erfindung die Aufgabe zugrunde, ein Operationsmikroskop der Eingangs genannten Art weiterzubilden, um die gewünschte Optimierung weiter zu vervollkommnen. Insbesondere soll ein Operationsmikroskop bereitgestellt werden, mittels dessen die oben beschriebenen störenden Reflexe unterdrückt werden können.

Diese Aufgabe wird erfindungsgemäß gelöst durch das Operationsmikroskop mit den Merkmalen gemäß dem unabhängigen Patentanspruch 1. Weitere Vorteile, Merkmale, Details, Aspekte und Effekte der Erfindung ergeben sich aus den Unteransprüchen, der Beschreibung sowie den Zeichnungen.

Es wird eine Beleuchtungseinrichtung für ein einen, zwei oder mehr Beobachtungsstrahlengänge mit jeweils einem Beobachtungsstrahlbündel aufweisendes ophthalmologisches Operationsmikroskop bereitgestellt, aufweisend zumindest eine Lichtquelle zum Erzeugen wenigstens eines Beleuchtungsstrahlbündels zum Beleuchten eines zu beobachtenden Objekts, insbesondere eines zu beobachtenden Auges, wobei das wenigstens eine Beleuchtungsstrahlbündel koaxial zu einem Beobachtungsstrahlbündel verläuft. Die Beleuchtungseinrichtung weist eine Beleuchtungsoptik auf, die nach dem Köhlerschen Beleuchtungsprinzip aufgebaut ist, und in der wenigstens eine Reflexblende zum Abdecken von Reflexlicht, das von der Oberfläche eines Objektivelements abgestrahlt wird, vorgesehen ist.

Der Begriff "Abdecken" im Kontext der vorliegenden Erfindung bedeutet dabei, dass Reflexlicht, welches ansonsten von der Oberfläche eines Objektivelements abgestrahlt werden würde, vermieden wird. Dabei kann durch die Reflexblende das Entstehen von Reflexlicht und dessen Abstrahlung von der Oberfläche eines Objektivelements vorteilhaft vollständig verhindert, zumindest aber abgeschwächt werden.

Gemäß dem ersten Aspekt der Erfindung wird somit ein Operationsmikroskop, mit einem, zwei oder mehr Beobachtungsstrahlengängen mit jeweils einem Beobachtungsstrahlbündel und mit einer Beleuchtungseinrichtung, die zum Beleuchten eines zu beobachtenden Auges ausgebildet ist, bereitgestellt, das die Merkmale von Anspruch 1 besitzt. Durch die erfindungsgemäße Beleuchtungseinrichtung lässt sich insbesondere eine Reflexunterdrückung in einem koaxialen Beleuchtungssystem für ein ophthalmologisches Operationsmikroskop realisieren.

Mit der vorliegenden Erfindung wird eine echte koaxiale Beleuchtung geschaffen. Unter "koaxial" wird dabei generell eine achsnahe Beleuchtung verstanden. Dies schließt sowohl eine Beleuchtung unter exakt Null Grad als auch eine achsnahe Schrägbeleuchtung unter einem sehr kleinen Winkel mit ein. Somit sind von der vorliegenden Erfindung auch solche Lösungen mit geringen Abweichungen von der koaxialen Beleuchtungsanordnung mit umfasst.

Erfindungsgemäß ist die Beleuchtungseinrichtung für ein ophthalmologisches Operationsmikroskop vorgesehen.

Im einfachsten Fall ist ein einziges Beleuchtungsstrahlbündel vorgesehen, das einen einzigen Beleuchtungsspot erzeugt. Es sind jedoch auch Ausgestaltungsformen mit zwei oder mehr Beleuchtungsstrahlbündeln möglich, wobei dann jeweils ein Beleuchtungsstrahlbündel jeweils einen Beleuchtungsspot erzeugt. Im letztgenannten Fall stellen die Beleuchtungsstrahlbündel - in der Gesamtheit betrachtet - jeweils Beleuchtungsteilstrahlbündel dar.

Erfindungsgemäß ist weiterhin vorgesehen, dass die Beleuchtungseinrichtung eine in besonderer Weise ausgestaltete Beleuchtungsoptik aufweist. Diese Beleuchtungsoptik ist zunächst dadurch gekennzeichnet, dass sie nach dem Köhlerschen Beleuchtungsprinzip ausgebildet ist. Das Köhlersche Beleuchtungsprinzip an sich ist bereits bekannt. Überraschender Weise wurde nun herausgefunden, dass dessen Einsatz in der Beleuchtungseinrichtung dazu führt, dass die störenden Reflexe unterdrückt werden können.

Zunächst weist die Beleuchtungseinrichtung eine Lichtquelle auf. Dabei ist die Erfindung nicht auf bestimmte Ausgestaltungsformen für die Lichtquelle beschränkt. Einige nicht ausschließliche Beispiele werden im weiteren Verlauf der Beschreibung näher erläutert.

Die nach dem Köhlerschen Prinzip aufgebaute Beleuchtungsoptik weist zunächst einen Kollektor auf. Dieser Kollektor kann vorteilhaft aus einem oder mehreren einzelnen oder zusammengesetzten Linsenelement(en) bestehen. Weiterhin ist eine Aperturblende vorgesehen. Diese kann beispielsweise als fixe oder variable Blende ausgebildet sein. Durch den Kollektor wird die Lichtquelle in ein Zwischenbild (Aperturblende) abgebildet.

Darüber hinaus gehört zur Köhlerschen Beleuchtung ein Kondensor. Bei der erfindungsgemäßen Beleuchtungseinrichtung liegt das Bild der Lichtquelle vorteilhaft im Brennpunkt des Kondensors. Vorgesehen ist, dass der Kondensor ein weiter unten noch näher beschriebenes Objektivelement einschließt. In einen solchen Fall kann realisiert werden, dass das Zwischenbild der Lichtquelle nach unendlich abgebildet wird.

Wenn es sich bei dem zu beobachtenden Objekt um ein Auge handelt, kann mit der erfindungsgemäßen Beleuchtungseinrichtung die Cornea des Auges telezentrisch beleuchtet werden. Weiterhin bildet das Auge die Lichtquelle wieder auf der Retina ab. Dieses Licht wird dann an der roten Retina reflektiert und beleuchtet die zu operierende Augenlinse gleichmäßig, intensiv und kontrastreich.

Zu einer Köhlerschen Beleuchtung gehört schließlich auch eine Irisblende - auch Leuchtfeldblende genannt -, die vorteilhaft als fixe oder variable Blende ausgebildet sein kann. Durch die Lichtquelle wird die Leuchtfeldblende ausgeleuchtet. Diese wird vorteilhaft durch den Kondensorteil ohne Objektivelement nach unendlich abgebildet und dann vom Objektivelement auf das zu beobachtende Objekt, beispielsweise auf die Cornea eines zu untersuchenden Auges. Ein solcher Aufbau ermöglicht vorteilhaft eine eindeutig getrennte und definierte Abbildung von Leuchtfeldblende und Lichtquelle.

Die Beleuchtungseinrichtung weist wenigstens ein Objektivelement auf. Das Objektivelement ist ebenfalls als Objektivelement des Operationsmikroskops, insbesondere als dessen Hauptobjektiv, ausgebildet.

Erfindungsgemäß sind Mittel vorgesehen, um jeweils ein Beobachtungsstrahlbündel und ein Beleuchtungsstrahlbündel zu überlagern. Diese Mittel können auf unterschiedlichste Weise ausgestaltet sein.

Die Mittel zum Überlagern sind derart angeordnet sind, dass eine Überlagerung von Beobachtungsstrahlbündel und Beleuchtungsstrahlbündel oberhalb des Objektivelements erfolgt. Die Überlagerung von Beobachtungsstrahlbündel und Beleuchtungsstrahlbündel kann beispielsweise im parallelen Strahlengang über dem Hauptobjektiv erfolgen.

Wie oben schon ausgeführt wurde, ist die Erfindung nicht auf bestimmte Ausgestaltungstypen von "Überlagerungsmitteln" beschränkt. Beispielsweise können die Mittel zum Überlagern wenigstens ein optisches Element in Form eines Prismas und/oder einer Strahlteilerplatte und/oder eines Spiegels, etwa eines teildurchlässigen Spiegels und/oder eines durchbohrten Spiegels, aufweisen. Natürlich können die Mittel auch anders ausgestaltet sein, so dass die Erfindung nicht auf die genannten Beispiele beschränkt ist.

In einer vorteilhaften Ausführungsform wird die Beleuchtung zwischen dem Objektivelement und einem Vergrößerungssystem, etwa einem Pankraten, im Beobachtungsstrahlengang unter Einsatz einer Teilerplatte in den Beobachtungsstrahlengang eingespiegelt.

Das Beleuchtungslicht wird durch das Objektivelement, bei dem es sich um das Hauptobjektiv des Operationsmikroskops handelt, hindurch abgebildet, aber auch an seinen Oberflächen reflektiert.

Um dies zu vermeiden, ist in der Beleuchtungsoptik erfindungsgemäß wenigstens eine Reflexblende zum Vermeiden von Reflexlicht von der Oberfläche eines Objektivelements vorgesehen. Diese Blende kann beispielsweise als fixe oder variable Blende ausgebildet sein.

Teile des entstehenden Reflexlichts werden durch die Beobachtungsoptik des Operationsmikroskops in das Auge eines Beobachters abgebildet und führen somit zu störenden Aufhellungen im Bild.

Dieses Reflexlicht ist natürlich Teil des genutzten Beleuchtungslichts und steht somit in Relation zu diesem. Wird das Reflexlicht dann an einer Stelle in der Beleuchtungsoptik mit einer Reflexblende abgedeckt, blendet man auch Teile des genutzten Beleuchtungslichts aus. Die rechnerische Lokalisierung dieses störenden Reflexlichts zeigt Unterschiede in der Relation zu dem Beleuchtungs-Nutzlicht, und zwar abhängig vom Ort innerhalb der Beleuchtungsoptik. Je nach Lage der Reflexblende wird das Beleuchtungslicht deutlich oder geringfügig abgeschwächt.

Die Lichtquelle, beziehungsweise das Zwischenbild der Lichtquelle strahlt zum einen in einem definierten Winkelbereich ab. Das Licht, das aus der Lichtquelle austritt, divergiert. Je größer die Abstrahlapertur, umso größer wird mit zunehmendem Abstand zur Lichtquelle, beziehungsweise zum Lichtquellenbild, der Büschelquerschnitt des Lichtbündels.

Das störende Reflexlicht ist zum anderen aber abhängig vom Winkel, unter dem es auf das Objektivelement, beispielsweise das Hauptobjektiv fällt. Es wird ein begrenzter Winkelbereich des Reflexlichts von der Beobachtungsoptik des Operationsmikroskops erfasst.

Je weiter weg man sich in der Beleuchtungseinrichtung vom Zwischenbild der Lichtquelle befindet, umso weiter weg von der Beleuchtungsachse wandert das störende Reflexlicht im Beleuchtungsbüschelquerschnitt. Das bedeutet, die größeren Winkel der Abstrahlaperturen erzeugen das Störlicht. Je näher am Zwischenbild der Lichtquelle die Reflexblende angebracht wird, umso mehr wird der eigentlich nicht störende Aperturbereich mit abgedeckt.

Die optimale Anordnung der Reflexblende im Beleuchtungsstrahlengang stellt daher eine besondere Herausforderung dar. Erfindungsgemäß ist vorgesehen, dass die Reflexblende im Beleuchtungsstrahlengang vor den Überlagerungsmitteln angeordnet ist.

Der optimale Ort für eine Reflexblende liegt möglichst weit entfernt vom Zwischenbild der Lichtquelle. Dies ist vor den Überlagerungsmitteln, etwa der Teilerplatte. Beispielsweise kann vorgesehen sein, dass zumindest einzelne der zuvor beschriebenen verschiedenen Blenden als fixe Blenden, oder aber als variable Blenden ausgestaltet sind. Beispielsweise kann wenigstens eine Blende als diskrete Blende, als LCD (Liquid Crystal Display) Display, als DMD (Digital Mirror Device), als LCOS (Liquid Crystal On Silicon) oder als FLCOS (Ferroelectric Liquid Crystal ON Silicon) ausgebildet sein. Denkbar sind auch andere transmissive oder reflektive Displays.

Wie weiter oben ausgeführt wurde, ist die Erfindung nicht auf eine bestimmte Anzahl von Lichtquellen oder deren Ausgestaltung beschränkt. Beispielsweise kann eine einzige Lichtquelle vorgesehen sein, die zunächst ein einziges Beleuchtungsstrahlbündel erzeugt. Beispielsweise kann auch vorgesehen sein, dass zwei oder mehr Lichtquellen vorgesehen sind und dass mittels jeder Lichtquelle ein Beleuchtungsstrahlbündel erzeugt wird. Es können somit unabhängige Lichtquellen verwendet werden, wobei jede Lichtquelle ein eigenes Beleuchtungsteilstrahlbündel erzeugt.

In einer anderen vorteilhaften Ausgestaltung kann vorgesehen sein, dass eine einzige Lichtquelle vorgesehen ist und dass Mittel zum Aufteilen des Beleuchtungsstrahlbündels der Lichtquelle in die zwei oder mehr Beleuchtungsteilstrahlbündel vorgesehen sind. Hierbei kann es sich um geeignete Strahlteiler in Form von Prismen, teildurchlässigen Spiegeln und dergleichen handeln.

Die vorliegende Erfindung ist nicht auf die Verwendung bestimmter Lichtquellen beschränkt. Nachfolgend werden hierzu einige nicht ausschließliche, vorteilhafte Beispiele genannt. Beispielsweise kann die wenigstens eine Lichtquelle als Lampe, insbesondere als Halogenlampe oder Xenonlampe, als Laser, als nicht thermischer Strahler, als Lichtleiter, insbesondere als Faserlichtleiterbündel, als wenigstens eine LED (Licht emittierende Diode), als wenigstens eine OLED (organische Licht emittierende Diode) oder dergleichen ausgebildet sein. Natürlich sind auch Kombinationen verschiedener Lichtquellen möglich.

Vorteilhaft ist die Lichtquelle aus einer Anordnung von einer oder mehreren einzeln oder bereichsweise schaltbaren Kleinstlichtquelle(n) gebildet. Die Beleuchtungseinrichtung ist dabei so ausgestaltet, dass sie bezüglich der von ihr erzeugten Leuchtfeldgeometrie einfach variiert werden kann. Dabei werden die Kleinstlichtquellen - insbesondere elektronisch - von außen, vorzugsweise von einer Steuereinrichtung, angesteuert. Ein weiteres Merkmal sieht vor, dass die Kleinstlichtquellen zumindest bereichsweise ansteuerbar sind, um variable Beleuchtungsgeometrien einstellen zu können. Dies ist insbesondere bei der Erzeugung von ringförmigen Beleuchtungsstrahlbündeln von Vorteil. Dabei ist die Erfindung nicht auf bestimmte Größen und/oder Formen von Bereichen beschränkt. Im einfachsten Fall kann ein einziger Punkt in solch einer Weise ansteuerbar sein. Insbesondere dann, wenn die Leuchtquelle aus einer Matrix bestehend aus einzelnen Kleinstlichtquellen gebildet ist, kann eine oder können mehrere Kleinstlichtquellen einzeln oder in Gruppen ansteuerbar sein, wobei im letztgenannten Fall einzelne Kleinstlichtquellen zu einem Bereich zusammengefasst werden können. Auch diesbezüglich ist die Erfindung nicht auf konkrete Ausgestaltungsformen beschränkt.

Vorteilhaft kann die Lichtquelle aus einer Anordnung von einer oder mehreren Leuchtdiode(n) (LED), insbesondere organischen Leuchtdiode(n) (OLED), gebildet sein. Organische Leuchtdioden sind ursprünglich als Mikrodisplays entwickelt worden. Anders als LCDs, die eine Hinterleuchtung benötigen, leuchten OLEDs selber als Lambertstrahler (Flächenemitter).

Als strukturierte Beleuchtungsquelle bieten OLEDs eine gute Lichteffizienz und kleine Strukturen ohne dunkle Zwischenräume. Entsprechend einer gewünschten Beleuchtungsgeometrie können einzelne der Kleinstlichtquellen angeschaltet werden und andere ausgeschaltet bleiben. Gegenüber LEDs ist bei OLEDs der Füllfaktor höher was bedeutet, dass eine höhere Packungsdichte realisierbar ist. Die Verwendung eines Displays aus LEDs oder OLEDs ermöglicht ein programmierbares, und beispielsweise auch automatisierbares Schalten unterschiedlicher Beleuchtungsmodi, ohne dass mechanische Komponenten, wie etwa Phasenkontrastringe, Filter, Abschwächer und dergleichen bewegt werden müssten. Besonders geeignet sind beispielsweise weiße OLEDs, deren Spektrum durch eine Mischung von organischen Molekülen bestimmt wird.

Durch die zuvor beschriebene erfindungsgemäße Beleuchtungseinrichtung können eine Reihe von Vorteilen realisiert werden. Zum einen entsteht kein Lichtverlust im Beobachtungsstrahlengang. Weiterhin kommt es nicht zu Vignettierungen oder Bildausfällen durch Blenden im Beobachtungsstrahlengang. Darüber hinaus sind auch keine aufwändigen Objektive erforderlich.

Grundmerkmal der erfindungsgemäßen Beleuchtungseinrichtung ist, dass die Beleuchtungsoptik nach dem Köhlerschen Beleuchtungsprinzip aufgebaut ist. Ein weiteres Merkmal besteht darin, dass wenigstens eine geeignete Blende in Form einer Reflexblende im Beleuchtungsstrahlengang vorgesehen ist.

Das Operationsmikroskop weist ein Hauptobjektivelement auf, welches identisch mit einem Objektivelement der Beleuchtungseinrichtung ist. Die Mittel zum Überlagern sind derart angeordnet, dass eine Überlagerung von Beobachtungsstrahlbündel und Beleuchtungsstrahlbündel oberhalb des Hauptobjektivelements erfolgt.

In anderer Ausgestaltung kann vorgesehen sein, dass das Operationsmikroskop ein Hauptobjektivelement aufweist, welches identisch mit einem Objektivelement der Beleuchtungseinrichtung ist, und dass die Mittel zum Überlagern derart angeordnet sind, dass eine Überlagerung von Beobachtungsstrahlbündel und Beleuchtungsstrahlbündel unterhalb des Hauptobjektivelements erfolgt.

Die Radien des Hauptobjektivs bestimmen die Richtung des Reflexlichts und damit das durch die Beobachtungsoptik des Operationsmikroskops erfasste Reflexlicht. Je kürzer der Radius der oberen Hauptobjektivfläche ist, desto weniger Reflexlicht fällt in die Beobachtungsoptik.

Eine Dezentrierung der Beobachtungskanäle, etwa der Beobachtungs-Stereokanäle, zum Hauptobjektiv beeinflusst ebenfalls das erfasste Reflexlicht. Je stärker die Beobachtungskanäle, etwa die Stereokanäle, zur Hauptobjektivachse dezentriert sind, umso weniger Reflexlicht wird erfasst.

Vorteilhaft ist daher vorgesehen, dass zwei oder mehr Beobachtungsstrahlengänge vorgesehen sind und dass die Beobachtungsstrahlengänge in Bezug auf das Hauptobjektivelement dezentriert ausgebildet sind.

Vorteilhaft kann das Operationsmikroskop als stereoskopisches Operationsmikroskop, insbesondere als Stereomikroskop, ausgebildet sein. Das optische System eines Operationsmikroskops besteht grundsätzlich aus mehreren Bauelementen, wie dem Tubus, dem Mikroskop-Grundkörper, usw. Zusätzlich ist es bei vielen Operationsmikroskopen möglich, unterschiedliche Zusatzmodule, wie zum Beispiel einen Mitbeobachtertubus für einen assistierenden Beobachter, eine Videokamera zur Dokumentation oder dergleichen anzuschließen.

Innerhalb des Mikroskop-Grundkörpers lassen sich wiederum mehrere Baugruppen zusammenfassen, wie beispielsweise eine Beleuchtungseinrichtung, eine Vergrößerungseinrichtung, das Hauptobjektiv oder dergleichen. Die charakteristische Größe beim Hauptobjektiv ist seine Brennweite, die den Arbeitsabstand vom Operationsmikroskop zum Operationsfeld festlegt und auch Einfluss auf die Gesamtvergrößerung des Mikroskops hat.

Vorzugsweise kann in dem wenigstens einen Beobachtungsstrahlengang ein Vergrößerungssystem vorgesehen sein. Hierbei kann es sich beispielsweise um einen Vergrößerungswechsler handeln, mit dem sich unterschiedliche Vergrößerungen einstellen lassen. In vielen Anwendungsfällen ist ein Vergrößerungswechsel in Stufen völlig ausreichend. Es ist jedoch auch möglich, als Vergrößerungssystem auch pankratische Vergrößerungssysteme zu verwenden, mittels derer eine stufenlose Vergrößerung (Zoomsystem) möglich ist.

Dabei kann vorteilhaft vorgesehen sein, dass die weiter oben bereits beschriebene Gerätepupille des Operationsmikroskops in dem Vergrößerungssystem angesiedelt ist.

Weiterhin können in dem wenigstens einen Beobachtungsstrahlengang ein Tubuselement und ein Okularelement vorgesehen sein. Die Aufgabe eines Okularelements ist generell die Nachvergrößerung des im Tubus entstehenden Zwischenbilds, sowie möglicherweise der Ausgleich eventueller Fehlsichtigkeiten des Nutzers eines solchen Mikroskops.

Vorteilhaft ist weiterhin vorgesehen, dass die Objektebene des zu untersuchenden Objekts im vorderen Brennpunkt des Hauptobjektivs ausgebildet ist. Dadurch wird erreicht, dass das zu untersuchende Objekt durch das Hauptobjektiv nach Unendlich abgebildet wird.

Vorteilhafterweise kann das Operationsmikroskop als stereoskopisches Operationsmikroskop, insbesondere als Stereomikroskop, ausgebildet sein. In diesem Fall verfügt das Operationsmikroskop über zwei parallel verlaufende Beobachtungsstrahlengänge.

Bei dem Operationsmikroskop kann es sich gemäß einer bevorzugten Ausführungsform um ein Stereomikroskop nach dem Teleskopprinzip handeln, das im Wesentlichen aus den drei optischen Teilkomponenten, nämlich Hauptobjektiv (afokales) Zoomsystem sowie binokulares Fernrohr aus Tubus und Okularen, besteht.

Zwischen den einzelnen Teilkomponenten des Operationsmikroskops verlaufen die Beobachtungsstrahlenbündel vorzugsweise parallel, sodass die einzelnen Teilkomponenten modular austauschbar und kombinierbar sind.

Erfindungsgemäß wird eine wie vorstehend beschriebene erfindungsgemäße Beleuchtungseinrichtung in einem ophthalmologischen Operationsmikroskop, vorzugsweise in einem für die Kataraktextraktion ausgebildeten Operationsmikroskop, verwendet. Ebenso kann ein wie vorstehend beschriebenes erfindungsgemäßes Operationsmikroskop als ein für die Kataraktextraktion ausgebildetes Operationsmikroskop verwendet werden.

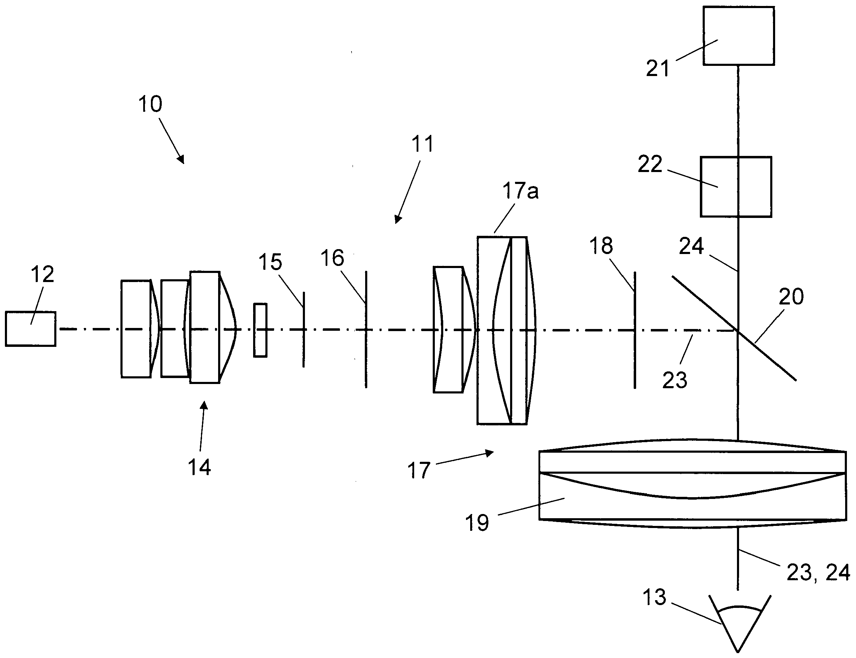

Die Erfindung wird nun anhand eines Ausführungsbeispiels unter Bezugnahme auf die beiliegende Zeichnung näher erläutert. Dabei zeigt die einzige Figur in schematischer Darstellung eine erfindungsgemäße Beleuchtungseinrichtung für ein ophthalmologisches Operationsmikroskop.

In der Figur ist eine Beleuchtungseinrichtung 10 dargestellt, die in einer als ophthalmologisches Operationsmikroskop, insbesondere als Stereo-Mikroskop, ausgebildeten Beobachtungseinrichtung zum Einsatz kommen kann.

Die Beleuchtungseinrichtung 10 weist eine Beleuchtungsoptik 11 auf, die nach dem Köhlerschen Beleuchtungsprinzip aufgebaut ist. Zunächst ist eine Lichtquelle 12 vorgesehen, beispielsweise eine Lichtfaser oder ein Bündel aus Lichtfasern. Das Faserende einer oder mehrerer Lichtfaser(n) wird durch einen Kollektor 14, der aus einem oder mehreren Linsenelementen, jeweils bestehend aus einem oder mehreren Linsengliedern, besteht, in ein Zwischenbild in einer Aperturblende 16 abgebildet.

Dieses Bild der Lichtquelle 12 liegt im Brennpunkt eines Kondensors 17. Der Kondensor 17 besteht im vorliegenden Beispiel aus einer Anzahl von Kondensor-Linsenelementen 17a. Weiterhin schließt er has Hauptobjektiv des Operationsmikroskops mit ein, bei dem es sich gleichzeitig auch um ein Objektivelement 19 für die Beleuchtungseinrichtung 10 handelt. Dadurch wird das Zwischenbild der Lichtquelle 12 nach unendlich abgebildet.

Bei dem zu beobachtenden Objekt 13 soll es sich im vorliegenden Beispiel um ein Auge handeln. Durch die vorstehend beschriebene Ausgestaltung wird die Cornea des zu untersuchenden Auges 13 telezentrisch beleuchtet, und das Auge 13 bildet die Lichtquelle 12 wieder auf der Retina ab. Dieses Licht wird an der roten Retina reflektiert und beleuchtet beispielsweise die zu operierende Augenlinse des Patienten gleichmäßig, intensiv und kontrastreich.

Die Lichtquelle 12 der Beleuchtungseinrichtung 10 leuchtet gleichzeitig eine Leuchtfeldblende 15 aus. Diese wird durch den Kondensorteil 17a ohne Hauptobjektiv 19 nach unendlich abgebildet und dann vom Hauptobjektiv 19 auf die Cornea des Auges 13.

Dieser Aufbau der Beleuchtungsoptik 11 ermöglicht eine eindeutig getrennte und definierte Abbildung von Leuchtfeldblende 15 und Lichtquelle 12.

Die Beleuchtung wird zwischen Hauptobjektiv 19 und einer Vergrößerungseinrichtung 22 des Operationsmikroskops, etwa einem Pankraten, durch Mittel 20 zum Überlagern von Strahlenbündeln, beispielsweise durch eine Teilerplatte, in den Beobachtungsstrahlengang 24 eingespiegelt. Zur Beobachtung ist eine im vorliegenden Beispiel nicht näher erläuterte Beobachtungsoptik 21 vorgesehen, die beispielsweise einen Tubus, Okularelemente und dergleichen umfassen kann.

Vom Beobachtungsstrahlengang ist zur Vereinfachung nur die Beobachtungsachse 24 dargestellt. Ebenso ist vom Beleuchtungsstrahlengang zur Vereinfachung nur die Beleuchtungsachse 23 dargestellt.

Das Beleuchtungslicht wird durch das Hauptobjektiv 19 hindurch abgebildet, aber auch an dessen Oberflächen reflektiert. Teile dieses Reflexlichts werden durch die Beobachtungsoptik 21 ins Beobachterauge abgebildet und führen somit zu störenden Aufhellungen im Bild.

Dieses Reflexlicht ist natürlich Teil des genutzten Beleuchtungslichts und steht somit in Relation zu diesem. Wird das Reflexlicht dann an einer Stelle in der Beleuchtungsoptik 11 mit einer Reflexblende 18 abgedeckt, blendet man auch Teile des genutzten Beleuchtungslichts aus. Die rechnerische Lokalisierung dieses störenden Reflexlichts zeigt Unterschiede in der Relation zu dem Beleuchtungs-Nutzlicht, und zwar abhängig vom Ort innerhalb der Beleuchtungsoptik. Je nach Lage der Reflexblende 18 wird das Beleuchtungslicht deutlich oder geringfügig abgeschwächt.

Die Lichtquelle 12, beziehungsweise das Zwischenbild der Lichtquelle 12 strahlt zum einen in einem definierten Winkelbereich ab. Das Licht, das aus der Lichtquelle 12 austritt, divergiert. Je größer die Abstrahlapertur, umso größer wird mit zunehmendem Abstand zur Lichtquelle 12, beziehungsweise zum Lichtquellenbild der Büschelquerschnitt des Lichtbündels.

Das störende Reflexlicht ist zum anderen aber abhängig vom Winkel, unter dem es auf das Hauptobjektiv 19 fällt. Es wird ein begrenzter Winkelbereich des Reflexlichts von der Beobachtungsoptik 21 erfasst.

Je weiter weg man sich in der Beleuchtungseinrichtung 10 vom Zwischenbild der Lichtquelle 12 befindet, umso weiter weg von der Beleuchtungsachse 23 wandert das störende Reflexlicht im Beleuchtungsbüschelquerschnitt. Das bedeutet, die größeren Winkel der Abstrahlaperturen erzeugen das Störlicht. Je näher am Zwischenbild der Lichtquelle 12 die Reflexblende 18 angebracht wird, umso mehr wird der eigentlich nicht störende Aperturbereich mit abgedeckt.

Der optimale Ort für eine Reflexblende 18 liegt somit möglichst weit entfernt vom Zwischenbild der Lichtquelle 12. Dies ist bei der erfindungsgemäßen Beleuchtungseinrichtung 10 vor den Überlagerungsmitteln 20, etwa der Teilerplatte. Eine Reflexblende 18 auf den Überlagerungsmitteln 20, beziehungsweise im Beobachtungsstrahlengang zwischen Vergrößerungseinrichtung 22 und Hauptobjektiv 19 würde Beobachtungsstrahlbüschel ausblenden.

Die Radien des Hauptobjektivs 19 bestimmen die Richtung des Reflexlichts und damit das durch die Beobachtungsoptik 21 erfasste Reflexlicht. Je kürzer der Radius der oberen Hauptobjektivfläche ist, desto weniger Reflexlicht fällt in die Beobachtungsoptik 21.

Eine Dezentrierung der Beobachtungskanäle, etwa der Beobachtungs-Stereokanäle, zum Hauptobjektiv 19 beeinflusst ebenfalls das erfasste Reflexlicht. Je stärker die Beobachtungskanäle, etwa die Stereokanäle, zur Hauptobjektivachse dezentriert sind, umso weniger Reflexlicht wird erfasst.

### Bezugszeichenliste

- 10: Beleuchtungseinrichtung
- 11: Beleuchtungsoptik
- 12: Lichtquelle
- 13: Zu beobachtendes Objekt
- 14: Kollektor
- 15: Leuchtfeldblende
- 16: Aperturblende
- 17: Kondensor
- 17a: Kondensor-Linsenelemente
- 18: Reflexblende
- 19: Objektivelement
- 20: Mittel zum Überlagern von Strahlbündeln
- 21: Beobachtungsoptik
- 22: Vergrößerungseinrichtung
- 23: Beleuchtungsachse
- 24: Beobachtungsachse

## Patentansprüche

1. Ophthalmologisches Operationsmikroskop, mit einem, zwei oder mehr Beobachtungsstrahlengängen mit jeweils einem Beobachtungsstrahlbündel und mit einer Beleuchtungseinrichtung (10), die zum Beleuchten eines zu beobachtenden Auges ausgebildet ist, aufweisend zumindest eine Lichtquelle (12), welche auf der Retina des Auges abgebildet wird, zum Erzeugen wenigstens eines Beleuchtungsstrahlbündels, wobei das wenigstens eine Beleuchtungsstrahlbündel koaxial zu einem Beobachtungsstrahlbündel verläuft, wobei das ophthalmologische Operationsmikroskop ein Objektivelement (19) aufweist, welches als Hauptobjektivelement des ophthalmologischen Operationsmikroskops ausgebildet und identisch mit einem Objektivelement der Beleuchtungseinrichtung (10) ist, wobei die Beleuchtungseinrichtung (10) eine Beleuchtungsoptik (11) aufweist, die Beleuchtungsoptik (11) nach dem Köhlerschen Beleuchtungsprinzip aufgebaut ist, wobei die Beleuchtungsoptik (11) einen Kollektor (14), eine erste Leuchtfeldblende (15) und eine zweite Aperturblende (16) aufweist, und wobei der Kollektor (14) ein Zwischenbild der Lichtquelle (12) in die Aperturblende (16) abbildet, und wobei die Beleuchtungsoptik (11) einen Kondensor (17) aufweist, welcher aus einer Anzahl von Kondensor-Linsenelementen (17a) besteht, das Hauptobjektivelement mit einschließt und das Zwischenbild der Lichtquelle (12) nach unendlich abbildet, wobei Überlagerungsmittel (20) vorhanden sind, um jeweils ein Beobachtungsstrahlbündel und ein Beleuchtungsstrahlbündel zu überlagern und diese Überlagerungsmittel (20) derart angeordnet sind, dass eine Überlagerung von Beobachtungsstrahlbündel und Beleuchtungsstrahlbündel auf der dem zu beobachtenden Auge abgewandten Seite des Hauptobjektivelements erfolgt, **dadurch gekennzeichnet, dass** in der Beleuchtungsoptik (11) wenigstens eine dritte Reflexblende (18) vorhanden ist, die das Entstehen von Reflexlicht und dessen Abstrahlung von der Oberfläche eines Objektivelements (19) in den Beobachtungsstrahlengang vollständig verhindert oder abschwächt, und dass die Reflexblende (18) im Beleuchtungsstrahlengang auf der der Lichtquelle zugewandten Seite des Objektivelements (19) zwischen den Kondensor-Linsenelementen (17a) und den Überlagerungsmitteln (20) vor den Überlagerungsmitteln (20) angeordnet ist.

2. Ophthalmologisches Operationsmikroskop nach Anspruch 1, **dadurch gekennzeichnet, dass** der Kollektor (14) aus einem oder mehreren einzelnen oder zusammengesetzten Linsenelement(en) besteht.

3. Ophthalmologisches Operationsmikroskop nach Anspruch 1 oder 2, **dadurch gekennzeichnet, dass** die Leuchtfeldblende (15) als fixe oder variable Blende ausgebildet ist.

4. Ophthalmologisches Operationsmikroskop nach einem der Ansprüche 1 bis 3, **dadurch gekennzeichnet, dass** die Aperturblende (16) als fixe oder variable Blende ausgebildet ist.

5. Ophthalmologisches Operationsmikroskop nach einem der Ansprüche 1 bis 4, **dadurch gekennzeichnet, dass** die Mittel (20) zum Überlagern wenigstens ein optisches Element in Form eines Prismas und/oder einer Strahlteilerplatte und/oder eines teildurchlässigen Spiegels und/oder eines durchbohrten Spiegels aufweisen.

6. Ophthalmologisches Operationsmikroskop nach einem der Ansprüche 1, 3 oder 4, **dadurch gekennzeichnet, dass** die Blende (15; 16; 18) als diskrete Blende, als LCD Display, als DMD, als LCOS oder als FLCOS ausgebildet ist.

7. Ophthalmologisches Operationsmikroskop nach einem der Ansprüche 1 bis 6, **dadurch gekennzeichnet, dass** zwei oder mehr Lichtquellen vorhanden sind und dass mittels jeder Lichtquelle ein Beleuchtungsstrahlbündel erzeugt wird.

8. Ophthalmologisches Operationsmikroskop nach einem der Ansprüche 1 bis 6, **dadurch gekennzeichnet, dass** eine einzige Lichtquelle (12) vorhanden ist und dass Mittel zum Aufteilen des Beleuchtungsstrahlbündels der Lichtquelle in zwei oder mehr Beleuchtungsteilstrahlbündel vorhanden sind.

9. Ophthalmologisches Operationsmikroskop nach einem der Ansprüche 1 bis 8, **dadurch gekennzeichnet, dass** die wenigstens eine Lichtquelle (12) als Lampe, insbesondere als Halogenlampe oder Xenonlampe, als Laser, als nicht thermischer Strahler, als Lichtleiter, insbesondere als Faserlichtleiterbündel, als wenigstens eine LED oder wenigstens eine OLED ausgebildet ist.

10. Ophthalmologisches Operationsmikroskop nach Anspruch 1, **dadurch gekennzeichnet, dass** zwei oder mehr Beobachtungsstrahlengänge vorhanden sind und dass die Beobachtungsstrahlengänge in Bezug auf das Hauptobjektivelement dezentriert ausgebildet sind.

## Claims

1. Ophthalmological surgical microscope, having one, two, or more observation beam paths with in each case one observation beam and having an illumination device (10) which is configured for illuminating an eye to be observed and has at least one light source (12) that is imaged on the retina of the eye for producing at least one illumination beam, wherein the at least one illumination beam extends coaxially with respect to an observation beam, wherein the ophthalmological surgical microscope has an objective element (19) which is configured as a main objective element of the ophthalmological surgical microscope and is identical to an objective element of the illumination device (10), wherein the illumination device (10) has an illumination optical unit (11), the illumination optical unit (11) is set up in accordance with the Köhler illumination principle, wherein the illumination optical unit (10) has a collector (14), a first field stop (15) and a second aperture stop (16), and wherein the collector (14) images an intermediate image of the light source (12) into the aperture stop (16), and wherein the illumination optical unit (11) has a condenser (17) which consists of a number of condenser lens elements (17a), also includes the main objective element and images the intermediate image of the light source (12) to infinity, wherein superposition means (20) are present to superpose in each case one observation beam and one illumination beam, and said superposition means (20) are arranged such that a superposition of observation beam and illumination beam is effected on the side of the main objective element that is remote from the eye to be observed, **characterized in that** at least one third reflection stop (18) is present in the illumination optical unit (11), said reflection stop (18) completely preventing or diminishing the formation of reflection light and the emission thereof from the surface of an objective element (19) into the observation beam path, and **in that** the reflection stop (18) is arranged in the illumination beam path on the side of the objective element (19) that is facing the light source between the condenser lens elements (17a) and the superposition means (20) upstream of the superposition means (20).

2. Ophthalmological surgical microscope according to Claim 1, **characterized in that** the collector (14) consists of one or more individual or assembled lens element(s).

3. Ophthalmological surgical microscope according to Claim 1 or 2, **characterized in that** the field stop (15) is configured as a fixed or variable stop.

4. Ophthalmological surgical microscope according to one of Claims 1 to 3, **characterized in that** the aperture stop (16) is configured as a fixed or variable stop.

5. Ophthalmological surgical microscope according to one of Claims 1 to 4, **characterized in that** the means (20) for superposition have at least one optical element in the form of a prism and/or a plate beam splitter and/or a partially transmissive mirror and/or a drilled mirror.

6. Ophthalmological surgical microscope according to one of Claims 1, 3 or 4, **characterized in that** the stop (15; 16; 18) is configured as a discrete stop, as an LCD display, as a DMD, as an LCOS or an FLCOS.

7. Ophthalmological surgical microscope according to one of Claims 1 to 6, **characterized in that** two or more light sources are present and **in that** an illumination beam is produced using each light source.

8. Ophthalmological surgical microscope according to one of Claims 1 to 6, **characterized in that** a single light source (12) is present and **in that** means for splitting the illumination beam from the light source in two or more illumination beams are present.

9. Ophthalmological surgical microscope according to one of Claims 1 to 8, **characterized in that** the at least one light source (12) is configured as a lamp, in particular a halogen lamp or xenon lamp, as a laser, as a non-thermal emitter, as a light guide, in particular as a fibre light guide bundle, as at least one LED or at least one OLED.

10. Ophthalmological surgical microscope according to Claim 1, **characterized in that** two or more observation beam paths are present and **in that** the observation beam paths are configured in a decentred manner with respect to the main objective element.

## Revendications

1. Microscope d'opération ophtalmologique, comprenant un ou deux trajets de rayons d'observation ou plus ayant respectivement un faisceau de rayons d'observation et comprenant un dispositif d'éclairage (10) qui est configuré pour éclairer un oeil à observer, possédant au moins une source de lumière (12), laquelle est représentée sur la rétine de l'oeil, servant à générer au moins un faisceau de rayons d'éclairage, l'au moins un faisceau de rayons d'éclairage se propageant de manière coaxiale par rapport à un faisceau de rayons d'observation, le microscope d'opération ophtalmologique possédant un élément formant objectif (19) qui est réalisé sous la forme d'un élément formant objectif principal du microscope d'opération ophtalmologique et qui est identique à un élément formant objectif du dispositif d'éclairage (10), le dispositif d'éclairage (10) possédant une optique d'éclairage (11), l'optique d'éclairage (11) étant construite selon le principe d'éclairage de Köhler, l'optique d'éclairage (11) possédant un collecteur (14), un premier diaphragme de champ lumineux (15) et un deuxième diaphragme d'ouverture (16), et le collecteur (14) représentant une image intermédiaire de la source de lumière (12) dans le diaphragme d'ouverture (16), et l'optique d'éclairage (11) possédant un condenseur (17) qui se compose d'une pluralité d'éléments formant lentille de condenseur (17a), inclut l'élément formant objectif principal et représente l'image intermédiaire de la source de lumière (12) vers l'infini, des moyens de superposition (20) étant présents pour superposer respectivement un faisceau de rayons d'observation et un faisceau de rayons d'éclairage et ces moyens de superposition (20) étant disposés de telle sorte qu'une superposition du faisceau de rayons d'observation et du faisceau de rayons d'éclairage s'effectue sur le côté de l'élément formant objectif principal à l'opposé de l'oeil à observer, **caractérisé en ce qu'**au moins un troisième diaphragme de réflexion (18) se trouve dans l'optique d'éclairage (11), lequel empêche complètement ou atténue l'apparition de lumière réfléchie et de son rayonnement depuis la surface d'un élément formant objectif (19) dans le trajet de rayons d'observation, et **en ce que** le diaphragme de réflexion (18) est disposé avant les moyens de superposition (20) dans le trajet de rayons d'éclairage sur le côté de l'élément formant objectif (19) faisant face à la source de lumière entre les éléments formant lentille de condenseur (17a) et les moyens de superposition (20).

2. Microscope d'opération ophtalmologique selon la revendication 1, **caractérisé en ce que** le collecteur (14) se compose d'un ou plusieurs éléments formant lentille individuels ou assemblés.

3. Microscope d'opération ophtalmologique selon la revendication 1 ou 2, **caractérisé en ce que** le diaphragme de champ lumineux (15) est réalisé sous la forme d'un diaphragme fixe ou variable.

4. Microscope d'opération ophtalmologique selon l'une des revendications 1 à 3, **caractérisé en ce que** le diaphragme d'ouverture (16) est réalisé sous la forme d'un diaphragme fixe ou variable.

5. Microscope d'opération ophtalmologique selon l'une des revendications 1 à 4, **caractérisé en ce que** les moyens (20) de superposition possèdent au moins un élément optique sous la forme d'un prisme et/ou d'une plaque séparatrice de faisceau et/ou d'un miroir partiellement transparent et/ou d'un miroir percé.

6. Microscope d'opération ophtalmologique selon l'une des revendications 1, 3 ou 4, **caractérisé en ce que** les diaphragmes (15 ; 16 ; 18) sont réalisés sous la forme de diaphragmes discrets, d'afficheurs LCD, de DMD, de LCOS ou de FLCOS.

7. Microscope d'opération ophtalmologique selon l'une des revendications 1 à 6, **caractérisé en ce que** deux sources de lumière ou plus sont présentes et **en ce qu'**un faisceau de rayons d'éclairage est généré au moyen de chaque source de lumière.

8. Microscope d'opération ophtalmologique selon l'une des revendications 1 à 6, **caractérisé en ce qu'**une source de lumière (12) unique est présente et qu'il existe des moyens pour diviser le faisceau de rayons d'éclairage de la source de lumière en deux faisceaux de rayons partiels d'éclairage ou plus.

9. Microscope d'opération ophtalmologique selon l'une des revendications 1 à 8, **caractérisé en ce que** l'au moins une source de lumière (12) est réalisée sous la forme d'une lampe, notamment d'une lampe halogène ou d'une lampe au xénon, sous la forme d'un laser, sous la forme d'un élément rayonnant non thermique, sous la forme d'un guide de lumière, notamment sous la forme d'un faisceau de fibres optiques, sous la forme d'au moins une LED ou d'au moins une OLED.

10. Microscope d'opération ophtalmologique selon la revendication 1, **caractérisé en ce que** deux trajets de rayons d'observation ou plus sont présents et **en ce que** les trajets de rayons d'observation sont représentés de manière décentrée par rapport à l'élément formant objectif principal.
